Europäisches Patentamt

European Patent Office

Office européen des brevets

(19)

(11) **EP 0 573 475 B1**

(12) **EUROPÄISCHE PATENTSCHRIFT**

(45) Veröffentlichungstag und Bekanntmachung des Hinweises auf die Patenterteilung:
**08.05.1996 Patentblatt 1996/19**

(21) Anmeldenummer: **92905167.0**

(22) Anmeldetag: **21.02.1992**

(51) Int Cl.$^6$: **C07C 57/15**, C07C 63/28, C07C 63/20, C07C 63/24, C07C 55/08

(86) Internationale Anmeldenummer:
**PCT/DE92/00143**

(87) Internationale Veröffentlichungsnummer:
**WO 92/15545 (17.09.1992 Gazette 1992/24)**

(54) **BASISCHE CALCIUM-ALUMINIUM-HYDROXID-DICARBOXYLATE, VERFAHREN ZU DEREN HERSTELLUNG UND DEREN VERWENDUNG**

BASIC CALCIUM ALUMINIUM HYDROXIDE DICARBOXYLATES, METHOD FOR PREPARING THEM AND USE THEREOF

DICARBOXYLATES BASIQUES D'HYDROXYDE D'ALUMINIUM ET DE CALCIUM, LEUR PROCEDE DE PRODUCTION ET LEUR UTILISATION

(84) Benannte Vertragsstaaten:
**AT BE DE ES FR GB IT NL**

(30) Priorität: **28.02.1991 DE 4106404**

(43) Veröffentlichungstag der Anmeldung:
**15.12.1993 Patentblatt 1993/50**

(73) Patentinhaber: **Bärlocher GmbH**
**D-80992 München (DE)**

(72) Erfinder:
• **RAZVAN, Coriolan**
 **D-8047 Karlsfeld (DE)**
• **BECK, Reinhard**
 **D-8000 München 60 (DE)**
• **KÜRZINGER, Alfred**
 **D-8047 Karlsfeld (DE)**
• **PÜRZER, Albert, W.**
 **D-8000 München 21 (DE)**
• **ROSENTHAL, Michael**
 **D-8000 München 40 (DE)**

(74) Vertreter: **Bezold, Gunter**
**Patentanwälte**
**Grünecker, Kinkeldey, Stockmair & Partner**
**Maximilianstrasse 58**
**80538 München (DE)**

(56) Entgegenhaltungen:
**WO-A-91/11421**

**Beschreibung**

Die Erfindung betrifft basische Calcium-Aluminium-Hydroxid-Dicarboxylate, ein Verfahren zu deren Herstellung und deren Verwendung als Stabilisatoren für halogenhaltige thermoplastische Harze, insbesondere Polyvinylchlorid.

Thermoplastische, halogenhaltige Harze, insbesondere PVC sind gegenüber Einwirkung von Wärme und Licht instabil. So tritt bereits bei der Verarbeitung von z. B. unstabilisiertem PVC ein thermischer Abbau des Harzes auf. Dies äußert sich in einer Verfärbung des Formteils und in der Verschlechterung der mechanischen Eigenschaften. Um diesen Nachteil auszuschließen, ist es notwendig, Wärmestabilisatoren in die Harzmasse einzuarbeiten. Hierzu werden üblicherweise organische und/oder anorganische Verbindungen der Metalle Blei, Barium, Cadmium, Calcium, Zinn und Zink alleine oder in Kombinationen zugegeben. Darüber hinaus werden noch andere Costabilisatoren wie Epoxide, organische Schwefelverbindungen, Polyole und Phosphite zugesetzt.

Zur Stabilisierung von PVC-Artikeln, wie Rohre, Platten, Profile und Kabelisolierungen werden bevorzugt basische Bleiverbindungen eingesetzt. Die am häufigsten verwendeten basischen Bleiverbindungen sind vom Sulfat-, Phosphit- oder Stearattyp.

Die DE-PS 12 19 223 und die DE-OS 24 19 379 lehren, daß PVC-Kabelisolierungen bevorzugt mit 2-basischem Bleiphthalat zu stabilisieren sind, da diese Verbindung dem Kabel hervorragende elektrische Eigenschaften verleiht.

In der EP-A- 0 313 113 wird erwähnt, daß 4-basisches Bleifumarat die wirksamste basische Bleiverbindung zur Stabilisierung von weicheingestellten halogenhaltigen Vinylpolymerisatmassen ist. Gemäß EP-A- 0 319 086 verleiht 5-basisches Bleifumarat PVC-Formteilen höhere Stabilität und einen besseren Weißgrad als andere bekannte Bleistabilisatoren.

Die organischen und/oder anorganischen Verbindungen der Schwermetalle Blei, Barium und Cadmium werden als toxisch eingestuft. Deshalb versucht man seit langem, diese durch nichttoxische Verbindungen zu ersetzen. Die als untoxisch betrachteten Stabilisatoren auf Basis von Kombinationen aus Calcium- und Zinkcarboxylaten sind in den meisten Anwendungsbereichen in ihrer Wirksamkeit unzureichend. Ihre Nachteile äußern sich in einer nichtausreichenden Langzeitstabilität und/oder einer unbefriedigenden Anfangsfarbe und Farbhaltung. Die Kombination dieser Metallseifen mit wirksamen Costabilisatoren, welche die Anfangsfarbe und die Langzeitstabilität verbessern, ist daher unerläßlich. So wird in der FR-A 2 403 362 beschrieben, Weich-PVC für Kabelisolierungen mit einer Mischung aus Calcium-Zink-Fettsäuren, Sorbit und einem β-Diketon zu stabilisieren. Die EP-A- 0256 872 beschreibt die Verwendung von Hydrotalkit und einem -Diketon zur Stabilisierung von PVC-Harzen. Es wurden auch Alkalialumosilikate in Verbindung mit anderen Costabilisatoren zur Verwendung in PVC vorgeschlagen (DE-A-31 13 442).

Alle bisher vorgeschlagenen untoxischen Stabilisierungssysteme haben jedoch Nachteile gegenüber schwermetallhaltigen Stabilisatoren. So erreichen sie meist nicht die erforderliche Langzeitstabilität. Eine gute Anfangsfarbe und ausreichende Farbhaltung können nur durch Einsatz von großen Mengen an teurem "Farbverbesserer" erreicht werden. Die metallhaltigen Costabilisatoren Hydrotalkit und Zeolith sind darin nachteilig, daß sie bei den für die Verarbeitung von z.B. PVC notwendigen Verarbeitungstemperaturen flüchtige Bestandteile abspalten, was zur Blasenbildung im Formteil führt. Ferner nehmen mit z.B. Polyol und/oder Zeolith stabilisierte PVC-Formteile Wasser auf, was zu erheblichen Problemen bei der weiteren Verarbeitung führt.

Die DE-A- 40 02 988 (Stand der Technik im Sinne von § 3 II PatG) beschreibt basische Calcium-Aluminium-Hydroxid-Dicarboxylate der Formel

$$Ca_xAl_2(OH)_{2(x+2)}A.m\ H_2O$$

worin bedeuten:

x   2 - 8;

m   0 - 12; und

A   ein aliphatisches, aromatisches oder heteroaromatisches Dicarbonsäureanion oder Kombinationen hiervon;

ein Verfahren zur deren Herstellung und deren Verwendung als Stabilisatoren für halogenhaltige thermoplastische Harze.

Der Erfindung liegt die Aufgabe zugrunde, neue Verbindungen sowie ein Verfahren zu deren Herstellung zur Verfügung zu stellen, die sich insbesondere als Stabilisator für halogenhaltige Polymere eignen, ohne die oben erwähnten Nachteile der bekannten Stabilisatoren aufzuweisen, insbesondere als nichttoxisch betrachtet werden.

Diese Aufgabe wird erfindungsgemäß einerseits gelöst durch die Bereitstellung basischer Calcium-Aluminium-Hydroxid-Dicarboxylate der allgemeinen Formel

$$Ca_xAl_2(OH)_{2(x+3-y)}A_y \cdot m\ H_2O$$

worin bedeuten:

x   2 -12;

$$\frac{2x+5}{2} > y > 0;$$

m   0 - 12; und

A   ein aliphatisches, aromatisches oder heteroaromatisches Dicarbonsäureanion oder Kombinationen hiervon.

ausgenommen, y = 1, wenn x = 2-8.

In der obigen Formel bedeutet x vorzugszweise 2 - 8, insbesondere bevorzugt 3 - 6 und m bedeutet vorzugsweise 2 - 4.

Die mit A angegebenen Dicarbonsäureanionen leiten sich beispielsweise ab aus Malonsäure, Bernsteinsäure, Adipinsäure, Fumarsäure, Maleinsäure, Phthalsäure, Isophthalsäure, Terephthalsäure und Pyridindicarbonsäuren. Das Fumarat- und Phthalatanion zählen zu bevorzugten Dicarbonsäureanionen.

Untersuchungen mittels Röntgenbeugung haben gezeigt, daß die erfindungsgemäßen Dicarboxylate hinsichtlich ihrer Kristallstruktur nicht dem Hydrotalkit-Typ angehören.

Überraschenderweise hat sich gezeigt, daß die erfindungsgemäßen Calcium-Aluminium-Hydroxid-Dicarboxylate halogenhaltigen, thermoplastischen Harzen und den daraus hergestellten Formteilen, vergleichbare Hitzestabilitäten wie basische Bleiverbindungen verleihen. Die Anfangsfarben und die Farbhaltung von z.B. Hart-PVC-Formteilen, die mit einer der neuen erfindungsgemäßen Verbindungen stabilisiert sind, sind den gleichen Formteilen, die bekannte nichttoxische Stabilisatorsysteme enthalten, gleichwertig.

Die der Erfindung zugrundeliegende Aufgabe wird andererseits gelöst durch ein Verfahren zur Herstellung der erfindungsgemäßen Calcium-Aluminium-Hydroxid-Dicarboxylate, das dadurch gekennzeichnet ist, daß man Mischungen aus Calciumhydroxid und/oder -oxid, Aluminiumhydroxid und Natriumhydroxid oder aus Calciumhydroxid und/oder -oxid und Natriumaluminat mit der entsprechenden Dicarbonsäure in zur Herstellung der erwünschten Verbindungen entsprechenden Mengen in wäßrigem Medium umsetzt und das Reaktionsprodukt in an sich bekannter Weise abtrennt und gewinnt. Das aus der oben beschriebenen Umsetzung direkt anfallende Reaktionsprodukt kann nach bekannten Verfahren vom wäßrigen Reaktionsmedium abgetrennt werden, vorzugsweise durch Filtration. Die Aufarbeitung des abgetrennten Reaktionsprodukts erfolgt ebenfalls in an sich bekannter Weise, beispielsweise durch Waschen des Filterkuchens mit Wasser und Trocknen des gewaschenen Rückstands bei Temperaturen von beispielsweise 60 - 130° C, vorzugsweise bei 90 - 120° C.

Für die Umsetzung kann sowohl ein feinteiliges, aktives Aluminiumhydroxid in Kombination mit Natriumhydroxid, als auch ein Natriumaluminat eingesetzt werden. Calcium kann in Form von feinteiligem Calciumoxid oder -hydroxid oder Mischungen daraus verwendet werden.

Die Umsetzungstemperaturen liegen vorzugsweise zwischen etwa 25 und 100° C, weiter vorzugsweise zwischen etwa 40 und 85° C. Katalysatoren oder Beschleuniger sind nicht erforderlich, können jedoch gegebenenfalls mitverwendet werden. Bei den erfindungsgemäßen Verbindungen kann das Kristallwasser ganz oder teilweise durch thermische Behandlung entfernt werden.

Bei ihrer Anwendung als Stabilisatoren spalten die erfindungsgemäßen, getrockneten Calcium-Aluminium-Hydroxid-Dicarboxylate bei den beispielsweise für Hart-PVC üblichen Verarbeitungstemperaturen von 160 - 200° C kein Wasser ab, so daß in den Formteilen keine störende Blasenbildung auftritt.

Zur Verbesserung ihrer Dispergierbarkeit in halogenhaltigen thermoplastischen Harzen könen die erfindungsgemäßen Verbindungen in bekannter Weise mit oberflächenaktiven Mitteln gecoatet werden.

Gemäß der Erfindung können mit den erfindungsgemäßen Calcium-Aluminium-Hydroxid-Dicarboxylaten halogenhaltige, thermoplastische Harze stabilisiert werden. Insbesondere eignen sich hierfür in bekannter Weise hergestellte Polyvinylchloride, Homo- und Copolymere davon sowie deren Abmischungen mit anderen Polymeren, wie z.B. ABS (Copolymer aus Acrylnitril/Butadien/Styrol), CPVC, (nachchloriertes PVC), Acrylate und dergleichen.

Zusätzlich zu den erfindungsgemäßen Verbindungen können selbstverständlich weitere Additive in das Harz eingearbeitet werden. Beispiele für solche Additive sind: Organozinnverbindungen, organische Phosphite, Epoxyverbindungen, Aminoverbindungen, mehrwertige Alkohole, Metallseifen von $C_8$ - $C_{22}$-Fettsäuren mit den Metallen Ca, Zn, Mg oder Al, Antioxidantien, UV-Absorber, Carbonylverbindungen, Antistatika, Gleitmittel, Weichmacher, Pigmente und Füllstoffe.

Die Erfindung wird durch die nachstehenden Beispiele näher erläutert.

A) Herstellung der erfindungsgemäßen, basischen Calcium-Aluminium-Hydroxid-Dicarboxylate.

<u>Beispiel 1</u>

Eine wäßrige Suspension (5,0 1) aus 222 g Calciumhydroxid (3 Mol) und 164 g Natriumaluminat (2 Mol) wird auf 50° C erwärmt. Anschließend werden 174 g Fumarsäure (1,5 Mol) in Form einer auf 85° C erwärmten, 10 % wäßrigen Lösung, mit gleichbleibender Zulaufgeschwindigkeit, im Laufe von 30 Minuten unter Rühren zugesetzt. Daraufhin wird die Suspension auf 70° C erwärmt und bei dieser Temperatur 2 Stunden gerührt. 10 Minuten vor Ende der Reaktionszeit werden 4 g Natriumstearat zur Coating zugegeben. Die so erhaltene Suspension wird abfiltriert und mit 1,8 l Wasser gewaschen. Der so entstandene Filterkuchen wird 4 Stunden bei 125° C im Trockenschrank getrocknet.

Die Analysenwerte des Produktes werden unten angegeben.

Molverhältnis

|  | Gefundener Wert | Berechneter Wert |
|---|---|---|
| Ca | 3,0 | 3,0 |
| Al | 1,9 | 2,0 |
| C | 2,2 | 2,0 |

<u>Beispiel 2</u>

Eine wäßrige Suspension (7,2 l) aus 444 g Calciumhydroxid (6 Mol), 80 g Natriumhydroxid (2 Mol) und 156 g aktives Aluminiumhydroxid (2 Mol) wird auf 70° C erwärmt. Anschließend werden 498 g Phthalsäure (3 Mol) in Form einer 8% wäßrigen Lösung (Temperatur 85° C) mit gleichbleibender Zulaufgeschwindigkeit im Laufe von 30 Minuten unter Rühren zugesetzt. Daraufhin wird die Suspension auf 80° C erwärmt und bei dieser Temperatur 2 Stunden gerührt. 10 Minuten vor Ende der Reaktionszeit werden 4 g Natriumstearat zur Coating zugegeben. Die so erhaltene Suspension wird abfiltriert, mit 2,3 1 Wasser gewaschen und der Filterkuchen in einem Trockenschrank bei 130° C 4 Stunden getrocknet. Die Analysenwerte des so hergestellten Produkts werden unten angegeben.

Molverhältnis

|  | Gefundener Wert | Berechneter Wert |
|---|---|---|
| Ca | 6,0 | 6,0 |
| Al | 2,1 | 2,0 |
| C | 16,4 | 16,0 |

B) Verwendung der erfindungsgemäßen Verbindungen als Stabilisatoren

In den folgenden Beispielen wird die Wärmestabilität und die Anfangsfarbe von PVC-Formkörpern, denen die erfindungsgemäßen Verbindungen zugesetzt worden sind, bewertet.

Für die Bewertung der Wärmestabilität werden die in den folgenden Beispielen verwendeten Mischungen auf einem Laborwalzwerk 5 Minuten bei 180° C homogenisiert und plastifiziert. Aus dem so hergestellten, etwa 1 mm dicken Fell werden quadratische Probeblättchen von 15 mm Kantenlänge geschnitten. Die Probeblättchen werden in einem Wärmeschrank bei 190° getempert. Im Abstand von 10 Minuten wird je ein Blättchen entnommen und auf einer Testkarte aufgeheftet. Dieser Vorgang wird so oft wiederholt, bis die Probeblättchen schwarz verfärbt sind.

**Beispiel 3**

|  | Gewichtsteile | | |
|---|---|---|---|
|  | A | B | C |
| PVC (K68) | 100 | 100 | 100 |
| Kreide | 6 | 6 | 6 |
| $TiO_2$ | 3 | 3 | 3 |
| Stearylstearat | 0,5 | 0,5 | 0,5 |
| Bisphenol A | 0,1 | 0,1 | 0,1 |
| Bleistearat | 1,0 | - | - |
| Dibasisches Bleiphthalat | 2,0 | - | - |
| Calciumstearat | 0,5 | 0,8 | 0,8 |
| Zinklaurat | - | 0,8 | 0,8 |
| $Ca_3Al_2(OH)_{11}(C_4H_2O_4)_{0,5}$ | - | 3,0 | - |
| $Ca_6Al_2(OH)_{14}(C_8H_4O_4)_{2,0}$ | - | - | 3,0 |
| Calciumacetylacetonat | - | 0,1 | 0,1 |

Die obigen Zusammensetzungen von A bis C wurden nach der angegebenen Methode getestet. Die Ergebnisse sind in der Tabelle I zusammengefaßt.

Tabelle I

| Ergebnisse der Bewertung der thermischen Stabilität | | | | | | | | | | | |
|---|---|---|---|---|---|---|---|---|---|---|---|
| Zeit (min.) | | | | | | | | | | | |
| Zusammensetzug | 0 | 10 | 20 | 30 | 40 | 60 | 80 | 100 | 120 | 140 | 160 |
| A | 1 | 1 | 1 | 1 | 3 | 3 | 3 | 3 | 3 | 5 | 5 |
| B | 1 | 1 | 1 | 1 | 2 | 2 | 2 | 4 | 4 | 4 | 6 |
| C | 1 | 1 | 1 | 2 | 2 | 2 | 2 | 4 | 4 | 4 | 6 |
| 1 = weiß | | 2 = schwach gelb | | 3 = schwach grau | | 4 = gelb | | 5 = grau | | 6 = braun | |

**Beispiel 4**

|  | Gewichtsteile | | |
|---|---|---|---|
|  | D | E | F |
| PVC K 70 | 100 | 100 | 100 |
| Kreide | 40 | 40 | 40 |
| $TiO_2$ | 2 | 2 | 2 |
| Di-iso-decylphthalat | 50 | 50 | 50 |
| Bisphenol A | 0,3 | 0,3 | 0,3 |
| Pentaerythrit | 0,1 | 0,1 | 0,1 |
| 2 basiche Bleiphthalat | 2,0 | - | - |
| Bleistearat | 1,0 | - | - |
| Calciumstearat | 0,5 | 0,8 | 0,8 |
| Zinklaurat | - | 0,8 | 0,8 |
| $Ca_3Al_2(OH)_{11}(C_4H_2O_4)_{0,5}$ | - | 3,0 | - |
| $Ca_6Al_2(OH)_{14}(C_8H_4O_4)_{2,0}$ | - | - | 3,0 |

Die obigen Zusammensetzungen von D bis F wurden nach der oben genannten Methode geprüft und die thermische Stabilität beurteilt. Die Ergebnisse sind in Tabelle II zusammengefaßt.

Tabelle II

| Zusammensetzug | Zeit (min.) | | | | | | | | | |
|---|---|---|---|---|---|---|---|---|---|---|
| | 0 | 10 | 20 | 40 | 60 | 80 | 120 | 160 | 180 | 200 |
| D | 1 | 1 | 1 | 1 | 1 | 3 | 3 | 3 | 3 | 5 |
| E | 1 | 1 | 1 | 1 | 1 | 2 | 2 | 4 | 4 | 6 |
| F | 1 | 1 | 1 | 1 | 2 | 2 | 2 | 4 | 4 | 6 |
| 1 = weiß | 2 = schwach gelb | | 3 = schwach grau | | 4 = gelb | | 5 = grau | | 6 = braun | |

**Patentansprüche**

**Patentansprüche für folgende Vertragsstaaten : AT, BE, DE, FR, GB, IT, NL**

1. Basische Calcium-Aluminium-Hydroxid-Dicarboxylate der allgemeinen Formel
$$Ca_xAl_2(OH)_{2(x+3=y)}A_y.m\ H_2O$$

   worin bedeuten:

   x   2 - 12;

   $$\frac{2x+5}{2} > y > 0;$$

   m   0 - 12; und

   A   ein aliphatisches, aromatisches oder heteroaromatisches Dicarbonsäureanion oder Kombinationen hiervon,

   ausgenommen y = 1, wenn x = 2-8.

2. Dicarboxylate nach Anspruch 1, worin x 2-8 bedeutet.

3. Dicarboxylate nach Anspruch 1, worin x 3 - 6 bedeutet.

4. Dicarboxylate nach Anspruch 1, 2 oder 3, worin m 2 - 4 bedeutet.

5. Dicarboxylate nach mindestens einem der Ansprüche 1 bis 4, worin A das Fumaratanion ist.

6. Dicarboxylate nach mindestens einem der Ansprüche 1 bis 4, worin A das Phthalatanion ist.

7. Verfahren zur Herstellung der basischen Calcium-Aluminium-Hydroxid-Dicarboxylate nach den Ansprüchen 1 bis 6, **dadurch gekennzeichnet**, daß man Mischungen aus Calciumhydroxid und/oder -oxid, Aluminiumhydroxid und Natriumhydroxid oder aus Calciumhydroxid und/oder -oxid und Natriumaluminat mit der entsprechenden Dicarbonsäure in zur Herstellung der erwünschten Verbindungen entsprechenden Mengen in wäßrigem Medium umsetzt und das Reaktionsprodukt in an sich bekannter Weise abtrennt und gewinnt.

8. Verfahren nach Anspruch 7, wobei die Umsetzung bei einer Temperatur zwischen etwa 25 und 100° C durchgeführt wird.

9. Verwendung der basischen Calcium-Aluminium-Hydroxid-Dicarboxylate gemäß den Ansprüchen 1 bis 6 als Stabilisatoren für halogenhaltige thermoplastische Harze, insbesondere Polyvinylchlorid.

**Patentansprüche für folgenden Vertragsstaat : ES**

1. Verfahren zur Herstellung von basischen Calcium-Aluminium-Hydroxi-Dicarboxylaten der allgemeinen Formel
$$Ca_xAl_2(OH)_{2(x+3-y)}A_y\cdot mH_2O$$

worin bedeuten:

x 2 -12;

$$\frac{2x+5}{2} > y > 0;$$

m 0 - 12; und

A ein aliphatisches, aromatisches oder heteroaromatisches Dicarbonsäureanion oder Kombinationen hiervon,

ausgenommen y = 1, wenn x = 2-8, dadurch gekennzeichnet, daß man Mischungen aus Calciumhydroxid und/oder -oxid, Aluminiumhydroxid und Natriumhydroxid oder aus Calciumhydroxid und/oder -oxid und Natriumaluminat mit der entsprechenden Dicarbonsäure in zur Herstellung der erwünschte Verbindungen entsprechenden Mengen in wäßrigem Medium umsetzt und das Reaktionsprodukt in an sich bekannter Weise abtrennt und gewinnt.

2. Verfahren nach Anspruch 1, wobei die Umsetzung bei einer Temperatur zwischen etwa 25 und 100°C durchgeführt wird.

3. Verfahren nach Anspruch 1 oder 2, worin x 2-8 bedeutet.

4. Verfahren nach Anspruch 1 oder 2, worin x 3-6 bedeutet.

5. Verfahren nach einem der Ansprüche 1 bis 4, worin m 2-4 bedeutet.

6. Verfahren nach einem der Ansprüche 1 bis 5, worin A das Fumaratanion ist.

7. Verfahren nach einem der Ansprüche 1 bis 5, worin A das Phthalatanion ist.

8. Verwendung der basischen Calcium-Aluminium-Hydroxid-Dicarboxylate, die gemäß den Ansprüchen 1 bis 7 erhalten werden, als Stabilisatoren für halogenhaltige thermoplastische Harze, insbesondere Polyvinylchlorid.

**Claims**

**Claims for the following Contracting States : AT, BE, DE, FR, GB, IT, NL**

1. Basic calcium/aluminium hydroxide dicarboxylates of the general formula

$$Ca_xAl_2(OH)_{2(x+3-y)}A_y \cdot mH_2O$$

in which:

x is 2 - 12;

$$\frac{2x+5}{2} > y > 0;$$

m is 0 - 12; and
A is an aliphatic, aromatic or heteroaromatic dicarboxylic acid anion or a combination thereof, but y cannot be 1 when x = 2 - 8.

2. Dicarboxylates according to Claim 1, in which x is 2 - 8.

3. Dicarboxylates according to Claim 1, in which x is 3 - 6.

4. Dicarboxylates according to Claim 1, 2 or 3, in which m is 2 - 4.

5. Dicarboxylates according to at least one of Claims 1 to 4, in which A is the fumarate anion.

6. Dicarboxylates according to at least one of Claims 1 to 4, in which A is the phthalate anion.

7. Process for the preparation of the basic calcium/ aluminium hydroxide dicarboxylates according to Claims 1 to 6, characterized in that mixtures of calcium hydroxide and/or oxide, aluminium hydroxide and sodium hydroxide or of calcium hydroxide and/or oxide and sodium aluminate are reacted with the corresponding dicarboxylic acid in appropriate amounts for the preparation of the desired compounds, in aqueous medium, and the reaction product is separated off and isolated in a manner known per se.

8. Process according to Claim 7, in which the reaction is carried out at a temperature between about 25 and 100°C.

9. Use of the basic calcium/aluminium hydroxide dicarboxylates according to Claims 1 to 6 as stabilizers for halogen-containing thermoplastic resins, in particular polyvinyl chloride.

**Claims for the following Contracting State : ES**

1. Process for the preparation of basic calcium/aluminium hydroxide dicarboxylates of the general formula

$$Ca_xAl_2(OH)_{2(x+3-y)}A_y \cdot mH_2O$$

in which:

x    is 2 - 12;

$$\frac{2x+5}{2} > y > 0;$$

m    is 0 - 12; and
A    is an aliphatic, aromatic or heteroaromatic dicarboxylic acid anion or a combination thereof, but y cannot be 1 when x = 2 - 8,

characterized in that mixtures of calcium hydroxide and/or oxide, aluminium hydroxide and sodium hydroxide or of calcium hydroxide and/or oxide and sodium aluminate are reacted with the corresponding dicarboxylic acid in appropriate amounts for the preparation of the desired compounds, in aqueous medium, and the reaction product is separated off and isolated in a manner known per se.

2. Process according to Claim 1, in which the reaction is carried out at a temperature between about 25 and 100°C.

3. Process according to Claim 1 or 2, in which x is 2 - 8.

4. Process according to Claim 1 or 2, in which x is 3 - 6.

5. Process according to one of Claims 1 to 4, in which m is 2 - 4.

6. Process according to one of Claims 1 to 5, in which A is the fumarate anion.

7. Process according to one of Claims 1 to 5, in which A is the phthalate anion.

8. Use of the basic calcium/aluminium hydroxide dicarboxylates obtained according to Claims 1 to 7 as stabilizers for halogen-containing thermoplastic resins, in particular polyvinyl chloride.

**Revendications**

**Revendications pour les Etats contractants suivants : AT, BE, DE, FR, GB, IT, NL**

1. Dicarboxylates d'hydroxyde de calcium et d'aluminium basiques répondant à la formule générale :

$$Ca_xAl_2(OH)_{2(x+3-y)}A_y \cdot m\,H_2O$$

dans laquelle :

x    = 2 - 12 ;

$$\frac{2x+5}{2} > y > 0;$$

m = 0 - 12 ; et

A désigne un anion d'acide dicarboxylique aliphatique, aromatique ou hétéroaromatique ou des associations de ceux-ci ;

excepté que y = 1 lorsque x = 2 - 8.

2. Dicarboxylates selon la revendication 1, dans lesquels x représente 2 - 8.

3. Dicarboxylates selon la revendication 1, dans lesquels x représente 3 - 6.

4. Dicarboxylates selon la revendication 1, 2 ou 3, dans lesquels m représente 2 - 4.

5. Dicarboxylates selon au moins une des revendications 1 à 4, dans lesquels A est l'anion fumarate.

6. Dicarboxylates selon au moins une des revendications 1 à 4, dans lesquels A est l'anion phtalate.

7. Procédé de préparation des dicarboxylates d'hydroxyde de calcium et d'aluminium basiques selon les revendications 1 à 6, caractérisé en ce qu'on fait réagir en milieu aqueux des mélanges d'hydroxyde et/ou d'oxyde de calcium, d'hydroxyde d'aluminium et d'hydroxyde de sodium ou d'hydroxyde et/ou d'oxyde de calcium et d'aluminate de sodium avec l'acide dicarboxylique approprié dans des quantités correspondant à la préparation des composés souhaités, et en ce qu'on sépare et obtient le produit de la réaction d'une manière connue en soi.

8. Procédé selon la revendication 7, dans lequel la réaction est effectuée à une température comprise entre environ 25 et 100 °C.

9. Utilisation des dicarboxylates d'hydroxyde de calcium et d'aluminium basiques selon les revendications 1 à 6 comme stabilisants pour des résines thermoplastiques halogénées, en particulier le polychlorure de vinyle.

**Revendications pour l'Etat contractant suivant : ES**

1. Procédé de préparation de dicarboxylates d'hydroxyde de calcium et d'aluminium basiques répondant à la formule générale :

$$Ca_xAl_2(OH)_{2(x+3-y)}A_y \cdot m\,H_2O$$

dans laquelle :

x = 2 - 12 ;

$$\frac{2x+5}{2} > y > 0;$$

m = 0 - 12 ; et

A désigne un anion d'acide dicarboxylique aliphatique, aromatique ou hétéroaromatique ou des associations de ceux-ci ;

excepté que y = 1, lorsque x = 2 - 8, caractérisé en ce qu'on fait réagir en milieu aqueux des mélanges d'hydroxyde et/ou d'oxyde de calcium, d'hydroxyde d'aluminium et d'hydroxyde de sodium ou d'hydroxyde et/ou d'oxyde de calcium et d'aluminate de sodium avec l'acide dicarboxylique approprié dans des quantités correspondant à la préparation des composés souhaités, et en ce qu'on sépare et obtient le produit de la réaction d'une manière connue en soi.

2. Procédé selon la revendication 1, dans lequel la réaction est effectuée à une température comprise entre environ 25 et 100 °C.

3. Procédé selcn la revendication 1 ou 2, dans lequel représente 2 - 8.

4. Procédé selon les revendications 1 ou 2, dans lequel représente 3 - 6.

**5.** Procédé selon les revendications 1 à 4, dans lequel m représente 2 - 4.

**6.** Procédé selon les revendications 1 à 5, dans lequel A est l'anion fumarate.

**7.** Procédé selon les revendications 1 à 5, dans lequel A est l'anion phtalate.

**8.** Utilisation des dicarboxylates d'hydroxyde de calcium et d'aluminium basiques qui sont obtenus selon les revendications 1 à 7, comme stabilisants pour des résines thermoplastiques halogénées, en particulier le polychlorure de vinyle.